# EUROPEAN PATENT APPLICATION

(11) **EP 1 752 534 A1**
(43) Date of publication of application: **14.02.2007**
(21) Application number: 05738527.0
(22) Date of filing: 12.05.2005
(51) Int. Cl.: C12N 15/09, C12Q 1/68, C12N 9/10

(54) **HIGH-SPEED PCR USING HIGH-SPEED DNA POLYMERASE**

(30) Priority: 18.05.2004 JP 2004147546
(71) Applicant: Toyo Boseki Kabushiki Kaisha, Osaka-shi, Osaka 530-8230 (JP)
(72) Inventor: SEGAWA, Masaya, c/o TOYO BOSEKI KABUSHIKI KAISHA, Tsuruga-shi, Fukui 914-0047 (JP); NAKAJIMA, Takashi, c/o TOYO BOSEKI K. K., Tsuruga-shi, Fukui 914-0047 (JP); OKA, Masanori, c/o TOYO BOSEKI KABUSHIKI KAISHA, Tsuruga-shi, Fukui 914-0047 (JP)
(74) Representative: Weber, Thomas
(86) International application number: PCT/JP2005/008674
(87) International publication number: WO 2005/111209

(57) **Abstract**

[PROBLEMS] To achieve a high-speed PCR which can be completed in a period of several minutes and thus can be used for a new application. [MEANS FOR SOLVING PROBLEMS] A method for practicing a high-speed PCR under a temperature change of 10°C/sec or more, **characterized in that** use is made of a heat-resistant DNA polymerase exhibiting a speed of synthesizing a deoxyribonucleic acid of 100 base/sec or higher, and a reagent kit for a high-speed PCR, or use in the method.

## Description

### TECHNICAL FIELD

The present invention relates to nucleic acid amplification techniques, polymerase chain reaction (PCR) in particular, that are important for the study of genes and its applications. The present invention is particularly useful for the analyses of gene expression, base polymorphism, or the like, and is applicable not only to research but has other applications as well, such as clinical diagnosis and environmental testing.

### BACKGROUND ART

Polymerase chain reaction (PCR) is a technique for amplifying specific sequences using two kinds of primers (see Non-Patent Publication 1, for example). Because the PCR has the sensitivity that allows for amplification from a nucleic acid sample as small as a single copy in principle and several copies in practice, and because of the specificity that can amplify only a specific portion, PCR has been used in a wide variety of fields including medical and biological researches and clinical diagnosis. As methods of detecting amplified nucleic acids, an electrophoresis method and a probe hybridization method have been widely used.

Non-Patent Publication 1: Science, 230, 1350-1354, 1985

With the spread of PCR, a wide variety of peripheral techniques have been developed. One example is a technique known as "hot start PCR." In this technique, modification is made to DNA polymerase so that the DNA polymerase, once deactivated, exhibits its activity at high temperatures. This is intended to suppress non-specific reactions at low temperatures, which occur during preparation of the reagents. Another technique is "real-time PCR," in which products of amplification are detected during the course of PCR, using fluorescent intercalaters or fluorescent probes. Despite these advances, reduction of a PCR reaction time--one of the most eagerly awaited improvements--has not been progressed at an expected pace.

PCR is performed in cycles each consisting of three steps: denaturation in which double-stranded DNA is separated by heat (about 90°C to 98°C); annealing in which the primers are bound to the single-stranded DNA (about 50°C to 70°C); and extension in which the complementary strands are synthesized from the primers (about 65°C to 75°C). Each cycle of amplification doubles a specific nucleic acid region flanked by the primers. In principle, 30 PCR cycles result in a 2³⁰ amplification. Though the reaction is simple, the reaction solution needs to be set at three different temperatures in each cycle. To this date, various techniques have been proposed to achieve such temperature changes. Among such techniques, the block method, which was employed in the early stage of development, is still in common use. In this technique, tubes with a reaction solution are inserted in a metal block, and a temperature change is achieved by heating the block. While the technique is structurally simple and desirable, a large heat capacity of the block makes it difficult to rapidly change the temperature. A high-speed has been achieved to some extent through modification of the Peltier element or reaction tube; however, it still takes about 2 hours to perform 40 PCR cycles.

Various techniques have been proposed to achieve a faster PCR. In the early days, there was proposed a method in which the reaction tube was mechanically transferred between water baths of three different temperatures. This method did not win popularity because it was prone to troubles such as vaporization or contamination of water, in addition to requiring a complex structure. There has also been a method that uses a liquid heat medium, or a method in which tubes are passed through blocks of three different temperatures. However, these methods never went beyond the planning stage owning to the complex structure they required. Under these circumstances, a method of temperature control using air has been put to actual applications as a high-speed PCR. Unlike the liquid heat medium, gas is free from the problem of vaporization or contamination. This method is employed by the Light Cycler of Roche Diagnostics, in which the temperature of glass capillaries containing a reaction solution is directly changed by hot air and cool air. The light cycler is considered to be the fastest among commercially available PCR devices. However, it still requires about 20 minutes for 40 PCR cycles. This is due to a small heat capacity of air.

Applicability of PCR would be greatly expanded if the speed of reaction were made faster. For example, in an environmental research of a possible pathogen-contaminated field, there is no time to waste depending on results. Further, measurement needs to be performed quickly in order to secure safety of the person conducting the measurement. In recent years, there has been proposed a technique in which spread of malignant tumors to the lymph node is diagnosed by genetic testing during the operation, so as to determine which portion should be excised. A rapid PCR would be highly useful also for these purposes. In order to meet these objects, PCR should be completed within at most 10 minutes.

Recently, Megabase of the United States has developed a device that allows for an even faster PCR (see Patent Publication 1, for example). This technique still uses the gas medium, but it uses a gas with a large heat capacity and attains an even greater heat capacity by increasing the gas pressure. Combined with fluid dynamics, it increases the heat conductivity for the glass capillaries containing the reaction solution. In this way, the device realizes a temperature change of 10°C/second or greater even in a 40 µL reaction system.
Patent Publication 1: USP 6,472,186

### BRIEF DESCRIPTION OF DRAWINGS

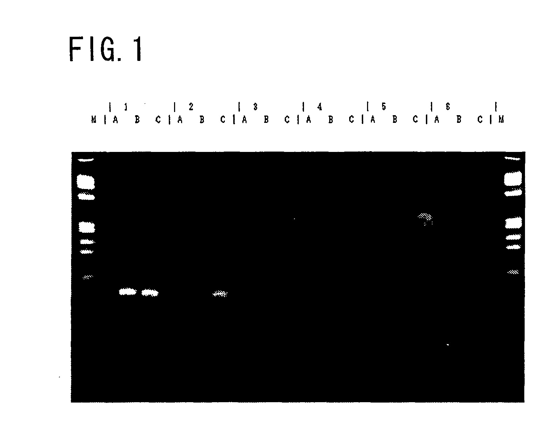

Figure 1 is a polyacrylamide gel electrophoretic image according to an Example of the present invention, in which the two lanes on the both ends are size markers (øX174, degraded by HaeIII), and sample lanes are denoted according to PCR cycle conditions (1-6) and PCR reaction solutions (A-C), as indicated from the left by Marker, 1-A, 1-B, 1-C, 2-A, 2-B, 2-C, 3-A, 3-B, 3-C, 4-A, 4-B, 4-C, 5-A, 5-B, 5-C, 6-A, 6-B, 6-C, and Marker.

### DISCLOSURE OF INVENTION

### PROBLEMS TO BE SOLVED BY THE INVENTION

With the temperature change as fast as the foregoing rate, the temperature change of the device no longer determines the reaction time of PCR. Rather, the reaction time is limited primarily by the PCR reaction itself. The inventors of the present invention looked into this further and found that while the denature step and the annealing step served their purposes when these steps had required temperatures even for a brief moment, the extension step required time according to the length of the amplified region. That is, the reaction time is limited by the reaction rate of DNA polymerase.

### MEANS TO SOLVE THE PROBLEMS

In this connection, the inventors of the present invention found that the reaction rate of DNA polymerase, and in particular the synthesis rate of deoxyribonucleic acid was important in high-speed PCR. The inventors diligently worked to find a solution to this end and accomplished the invention.

Specifically, according to the present invention, there are provided:

A method for performing a high-speed PCR under a temperature change of 10°C/sec or greater, the method comprising using a heat-resistant DNA polymerase having a deoxyribonucleic acid synthesis rate of 100 bases / sec or greater.

A method as set forth in claim 1, wherein the heat-resistant DNA polymerase originates in *Thermococcus.*

A method as set forth in claim 1, wherein the heat-resistant DNA polymerase originates in *Thermococcus kodakaraensis.*

A composition for high-speed PCR that is performed under a temperature change of 10°C/sec or greater, the composition comprising a heat-resistant DNA polymerase having a deoxyribonucleic acid synthesis rate of 100 bases / sec or greater.

A reagent kit for high-speed PCR that is performed under a temperature change of 10°C/sec or greater, the reagent kit comprising a heat-resistant DNA polymerase having a deoxyribonucleic acid synthesis rate of 100 bases / sec or greater.

### EFFECTS OF THE INVENTION

The present invention realizes a high-speed PCR that does not incur any additional cost or any inconvenience over conventional methods. A method according to the present invention will be economically very beneficial in a wide variety of fields where PCR is performed.

### BEST MODE FOR CARRYING OUT THE INVENTION

The following will describe the present invention in detail.

As used herein, "a temperature change during a high-speed PCR" refers to a change in average temperature over a total time period required for each step of temperature increase or decrease. A means to achieve the temperature change of 10°C/second or greater is not particularly limited. For example, the system described in USP 6,472,186 can be used.

The inventors of the present invention have been assessing ways to increase the speed of PCR using a prototype of high-speed PCR device (hereinafter, "PCRJet," the product of Megabase, US). In an attempt to reduce the time of one cycle, the inventors examined the time required for each step. It was found as a result that amplification still occurred even when the denature step was performed, for example, at 95°C for 0.2 seconds. This means that amplification occurs when the denature step has this temperature even for a brief moment. This contrasts to the annealing and extension steps, which require a certain amount of time. These observations have also been confirmed in the Examples of USP 6, 472, 186. The inventors of the present invention studied the annealing step and extension step further, and found that the rate determining process was the extension of DNA. More specifically, while it takes a relatively short time to physically bind (anneal) the primers to the complementary strands, extension of DNA from the annealed primers takes time. That is, even when a desirable amplification cycle has apparently distributed much of its time to the annealing step, what is really happening in the annealing step is the extension of DNA by the DNA polymerase.

There are several ways to reduce the time required for the extension step. The most effective approach is to shorten the amplified region of PCR as much as possible. This automatically reduces the time required for the extension. The amplified region cannot be shortened when the DNA contained therein is to be cloned (extracted) for research purposes. However, this is rather rare, and it is possible to shorten the amplified region in more common cases, for example, when confirming the presence or absence of bacteria or viruses, quantifying the expression level of mRNA, or determining single nucleotide polymorphisms (SNPs). Indeed, extremely short regions are amplified in PCR that is commonly performed these days for the quantification of mRNA or determination of SNPs. However, even in these cases, since each primer is about 20 to 30 mer, an amplified region of at least about 60 base pairs will still be required, including the primer portions. Further, when probes are used for detection purposes, an additional length needs to be provided. As a result, amplification of about 100 bp will be required. As set out above, there is a limit in reducing the length of the amplified region, and other approaches need to be sought for.

One possible approach is to increase the reaction rate. Denaturation and annealing cause physical separation or binding due to temperature changes, and as such the reactions proceed smoothly to completion. In contrast, extension inevitably takes time because it involves complex chemical reactions catalyzed by the enzyme, DNA polymerase. Having achieved the fast rate of temperature change, the last key to the shorter cycles is the reduction of the extension step. The time required for the extension step is determined by the length of amplified region and the rate of extension reaction.

The present invention was achieved by increasing the reaction rate.

Among the factors involved in improvement of the reaction rate, some examples include the type of DNA polymerase catalyzing the reaction, the compositions of enzymes and reagents directly involved in the reaction or used together with DNA polymerase, and other reaction conditions such as pH or temperature.

One of the most important factors is the type of DNA polymerase used to catalyze the reaction.

Generally, Taq DNA polymerase is used for PCR. Taq DNA polymerase originates in one kind of Eubacteria, *Thermus aquaticus,* and is suitable for PCR because it is heat resistant. Taq DNA polymerase is also used in the Examples of USP 6, 472, 186. The publication also uses a particular type of Taq DNA polymerase, "Z-Taq" (Takara Bio Inc.), which has been optimized for a fast extension reaction. In the assessment made by the inventors of the present invention, in a PCR of 75 bp using Taq DNA polymerase, the annealing step and the extension step required a total of at least 7 seconds, and a total of about 10 seconds to obtain distinct bands. This means that about 10 minutes are required for 40 cycles.

The synthesis rate of Taq DNA polymerase is about 60 bases/sec. Theoretically, amplification of 75 base pairs should complete in 1 second, instead of at least 7 seconds actually observed. This was considered to be due to processivity (the ability to synthesize continuously), and priming capability.

The inventors focused on this point, and after extensive research, found that the extension step could be reduced with use of high-performance DNA polymerase. Specifically, it was possible to clearly shorten the extension step when heat-resistant DNA polymerase was used that had a DNA synthesis rate of at least 100 bases/sec. The effect was pronounced when DNA polymerase originating in *Thermococcus* (hyperthermophilic Archaea) was used. Archaea is also known as archaebacteria, and it belongs to a group of organisms totally distinct from true bacteria, or commonly called simply "bacteria," are often found in an extreme environment of high pressure and heat, for example. Some produce enzymes of superior properties.

The inventors have found that the effect could be maximized with the DNA polymerase (hereinafter, "KOD DNA polymerase") originating in *Thermococcus kodakaraensis* isolated from solfatara. With KOD DNA polymerase, the annealing step and the extension step in the PCR of 75 base pairs required a total of at least 3 seconds, and a total of about 5 seconds to obtain distinct bands. That is, the time required for these steps was reduced in half compared with the case where Taq DNA polymerase was used. As a result, the inventors were able to realize a PCR that required a little more than 5 minutes for 40 cycles of reaction, thus creating a new area in the field of nucleic acid amplification.

Specifically, according to one embodiment of the present invention, there is provided a method for performing high-speed PCR under a temperature change of 10°C/second or greater, the method using a heat-resistant DNA polymerase having a deoxyribonucleic acid synthesis rate of 100 bases/second or greater.

As used herein, the synthesis rate for deoxyribonucleic acid as denoted by "bases/second" refers to the number of DNA synthesized per unit time. The following describes a method of measurement.

A DNA polymerase reaction solution (20 mM Tris-HCl buffer (pH 7.5), 8 mM magnesium chloride, 7.5 mM dithiothreitol, 100 µg/ml BSA, 0.1 mM dNTP, 0.2µCi[α-32P]dCTP) is allowed to react with M 13mp 18 single-stranded DNA to which the primers have been annealed. The reaction is stopped by adding the same amount of a reaction stop solution (50 mM sodium hydroxide, 10 mM EDTA, 5% Ficoll, 0.05% bromophenol blue). The DNA synthesized by the reaction is fractionated by alkali agarose gel electrophoresis, and the gel is dried for autoradiography. As a DNA size marker, labeled λ/HindIII is used. The band of the marker is used as an index to measure the size of synthesized DNA. From this, the synthesis rate of deoxyribonucleic acid is determined.

KOD DNA polymerase has the DNA synthesis rate greater than 100 to 140 bases/second, which doubles that of Taq DNA polymerase. The superior processivity may also account for the fast synthesis rate. Evidently, the fast DNA synthesis rate of KOD DNA polymerase is very useful for PCR that is designed to amplify long regions. In fact, the effect of reducing the extension step multiplies in a long-chain PCR. Therefore, the realization of high-speed PCR itself is industrially very useful.

KOD DNA polymerase used in the present invention is readily available from, for example, ToYo Boseki Kabushiki Kaisha (product code KOD-101). Further, a natural polymerase that has incorporated deletion, substitution, or addition of one or more amino acids (mutant) by known means may be used. Further, such enzymes (natural or mutant) may be modified with a chemical modifier, for example.

Note that, the heat-resistant DNA polymerase with the deoxyribonucleic acid synthesis rate of greater than 100 bases/second, as used in the present invention, is not just limited to KOD DNA polymerase, or a mutant or variant thereof.

Currently, no DNA polymerase (either individually or in combination) can provide a deoxyribonucleic acid synthesis rate of greater than 100 bases/second without any modification. Some of the examples of such conventional DNA polymerase include: Taq polymerase, EX-Taq, LA-Taq, Expand series, Platinum series, Tbr, Tfl, Tru, Tth, Tli, Tac, Tne, Tma, Tih, Tfi (all PolI enzymes), Pfu, Pfutubo, Pyrobest, Pwo, KOD, Bst, Sac, Sso, Poc, Pab, Mth, Pho, ES4, VENT, DEEPVENT (all α enzymes). In recent years, the primary structure and three-dimensional structure of such DNA polymerases have been revealed, and there has been accumulation of knowledge concerning relationships between structure and function, and similarities and differences of the enzymes of different origins. The present invention also encompasses DNA polymerases that have been mutated or modified based on such new knowledge to provide a deoxyribonucleic acid synthesis rate of greater than 100 bases/second, or that have been appropriately combined (including KOD DNA polymerase) to provide such synthesis rate.

It is preferable that the DNA polymerase be selected taking into account the synthesis rate, accuracy, processivity, priming capability, and heat-resistance altogether. For example, α enzyme provides good accuracy. One example of a method for evaluating the accuracy of DNA synthesis is the method using ribosomal protein S12 (rpsL) gene involved in streptomycin resistance. Streptomycin is an antibiotic that inhibits protein synthesis in prokaryotic cells. It binds to 30S ribosomal RNA (rRNA) of bacteria to inhibit the reaction forming an initiation complex of protein synthesis. It also causes translation error of the genetic code. A streptomycin-resistant mutant strain has mutation in ribosome protein S12. This mutation is known to have pleiotropic effect, such as suppressing the suppressor tRNA from reading the terminator codon, that improves the faithfulness of the translation by the ribosome. When PCR is performed for amplification using rpsL gene as a template, mutation occurs at certain probabilities. If the mutation occurs at amino acid level, the structure of rpsL protein is changed, with the result that streptomycin fails to act on 30S ribosomal RNA. Thus, when transforming *E. coli* with amplified plasmid DNA, streptomycin-resistant bacteria occur more frequently as the number of mutations is increased.

Plasmid pMol 21 (Journal of Molecular Biology (1999) 289, 835-850) includes rpsL gene and ampicillin-resistant gene. A PCR primer set (one of the primers was biotinylated, and MluI restriction enzyme site was introduced) for PCR amplification was designed on the ampicillin-resistant gene of the plasmid, and the entire length of the plasmid was amplified by PCR using a heat-resistant DNA polymerase. The product of PCR was purified with streptavidin beads, and after excision with restriction enzyme MluI, the DNA fragments were ligated by DNA ligase to transform the *E. coli.* The bacteria were inoculated on two kinds of plates, one containing ampicillin and one containing ampicillin and streptomycin. By calculating a ratio of colonies formed on the two plates, accuracy of DNA synthesis can be determined.

DNA polymerase is not the only factor that determines the reaction rate. Other important factors include the compositions of enzymes and reagents directly involved in the reaction or used together with DNA polymerase, and other reaction conditions such as pH or temperature. High-speed PCR generally requires more magnesium and primers than usual PCR, with the final magnesium concentration of no less than 3 mM, and the final primer concentration of no less than 400 nM.

According to one embodiment of the present invention, there is provided a method of synthesizing extensions of DNA primers with the use of DNA polymerase, wherein the primers are extended by the reaction between the primers and four kinds of dNTPs, using DNA as a template. The primers are two kinds of oligonucleotides, each preferably being complementary to the DNA generated by the other. It is also preferable that the method repeat a cycle of heating and cooling. In the present invention, it is preferable that, in order to sustain activity of DNA polymerase, PCR include divalent ions, for example, such as magnesium ion, and monovalent ions, for example, such as ammonium ion and/ or potassium ion. Further, in addition to a buffer and such ions (may be in the form of salts), the PCR reaction solution may also include BSA, a non-ionic surfactant such as Triton X-100, and buffer. The buffer may be a good buffer, for example, such as TRIS, TRICINE, BIS-TRICINE, HEPES, MOPS, TES, TAPS, PIPES, or CAPS. A phosphate buffer can be used as well.

In addition to the DNA synthetase, a reaction solution composition of the present invention may include forward and reverse primers with base sequences complementary to the target nucleic acid; four kinds of dNTPs; divalent cations; buffer; an intercalater fluorescent reagent such as SYBR and Green I; nucleic acid probes; a coloring reagent; and a luminescent reagent.

The type of nucleotide included in the present invention is not particularly limited. One example is a deoxyphosphonucleotide or its derivative. Specifically, the composition preferably includes a substance selected from the group consisting of: dATP, dCTP, dGTP, dTTP, dITP, dUTP, α-thio-dNTPs, biotin-dUTP, fluorescein-dUTP, and digoxigenin-dUTP, for example.

The type of salt included in the present invention is not particularly limited. For example, the salt may be one selected from the group consisting of: potassium chloride, potassium acetate, potassium sulfate, ammonium sulfate, ammonium chloride, ammonium acetate, magnesium chloride, magnesium acetate, magnesium sulfate, manganese chloride, manganese acetate, manganese sulfate, sodium chloride, sodium acetate, lithium chloride, and lithium acetate. These salts are readily available from commercial products.

Further, as required, the present invention preferably includes antibodies for suppressing polymerase activity and/or 3'-5' exonuclease activity of DNA polymerase, or production of by-products such as primer dimers, or for protecting the primers from degradation. Examples of such antibodies include a monoclonal antibody and a polyclonal antibody. Known enhancers may be included as well.

Further, the present invention may include substances, as listed below, that promote DNA synthesis.

Anionic substance. The type of anionic substance is not particularly limited. One example is a substance with a carboxyl group, preferably dicarboxylate. Dicarboxylate may be added in the form of an inorganic salt, for example. Preferably, at least one of oxalate ion, malonate ion, maleate ion is added. More preferably, such inorganic salts are in the form salts combined with an alkali metal (preferably, potassium salt or sodium salt), an alkali earth metal, or ammonium. Specific examples include: zinc oxalate; ammonium oxalate; potassium oxalate; calcium oxalate; diethyl oxalate; N'-N-disuccinimidyl oxalate; dimethyl oxalate; tin oxalate; cerium oxalate; iron oxalate; copper oxalate; sodium oxalate; nickel oxalate; bis oxalate; (2,4-dinitrophenyl) oxalate; (2,4,6-trichlorophenyl) oxalate; manganese oxalate; methyl oxalate; lanthanum oxalate; lithium oxalate; isopropylidene malonate; ethyl malonate; diethyl malonate; dibenzyl malonate; dimethyl malonate; thallium malonate; disodium malonate; monosodium maleate; diethyl maleate; chlorpheniramine maleate; di-n-butyl maleate; and mono-n-butylester maleate. All of these salts are commercially available. The anionic substance is usually used at a concentration of 0.1 mM to 20 mM, preferably 0.1 mM to 10 mM, and more preferably 1 mM to 10 mM.

A reagent including glycine as a basic unit. The type of such reagent is not particularly limited. Preferably trimethylglycine (betaine) is used, which is commercially available. The effective concentration of trimethyl glycine (betaine) is 0.5 M to 2 M, preferably 0.5 M to 1.5 M, and more preferably 1 M to 1.5 M.

DMSO. This is also commercially available. The effective concentration of DMSO is 0.1% to 15%, preferably 2% to 10%, and more preferably 5% to 10%.

The effects of the present invention can be enhanced by adding the anionic substance in appropriate combination with either a reagent including glycine as a basic unit, or DMSO.

The present invention is also applicable to a real-time PCR method.

A real-time PCR method includes a technique using various types of fluorescent probes, and a technique using an intercalater dye. The probe may be, for example, TaqMan (for example, see Japanese PCT Laid-Open Publication No. 2002-505071), Molecular Beacon, Hybridization Probe, or Cycling Probe. As an intercalater fluorescent dye, SYBR Green I (Molecular Probes, Inc.) is available. The intercalater method is particularly suitable in the present invention. Since the intercalater binds instantly, the intercalater method can be applied to the ultra high-speed PCR of the present invention without providing any wait time. In contrast, the probe method often requires an additional time for allowing the probes to bind. It should also be noted that not all probe techniques are applicable depending on exonuclease activity of the DNA polymerase.

For better accuracy and stability of assay results, real-time PCR sometimes uses a passive reference that is intended to correct variations in the amount of reaction solution used, or variations in the fluorescent characteristics of the vessels. As used herein, "passive" means that there is no significant change in the fluorescence of the reference molecules during the real-time PCR (see Japanese Patent No. 3454432, for example). When cost and time are of concern, a fluorescent material with a hydroxy group, for example, such as 5- and/or 6-carboxy X Rhodamine can be used (for example, available from Research Organics, Inc.). The inventors have confirmed that such products are usable in the present invention without causing any problem.

Further, the present invention is also applicable to a technique known as a hot start PCR method. The hot start PCR method is a technique whereby activity of DNA polymerase is suppressed until the first denaturation reaction, so that a so-called "non-specific reaction," such as the reaction on non-target nucleic acid sequences, or formation of primer dimers by the reaction between primers can be suppressed. The hot start PCR is performed, for example, by physically isolating the DNA polymerase until the first denaturation reaction, or by blocking the polymerase active domain or exonuclease active domain of the DNA polymerase with a chemical modifier or an anti-DNA polymerase antibody. These blocks come off by heat, allowing the DNA polymerase to exhibit activity after the first denaturation reaction. As a result, PCR proceeds. It should be noted here that the chemical modifier requires several minutes to remove the blocks. This cancels out the time afforded by the high-speed PCR of the present invention. In contrast, the antibody blocks require only several seconds to come off. This helps achieve the effects of the present invention more easily in terms of speed.

### Example

The following describes the present invention in more detail. It should be appreciated however that the invention is not limited in any way by the following description.
1. Example: Comparison between Taq DNA Polymerase and KOD DNA Polymerase in High-Speed PCR Synthesis of Primers for Detecting Human G3PDH Gene
   An oligonucleotide with the base sequence of SEQ ID NO: 1 (primer 1), and an oligonucleotide with the base sequence of SEQ ID NO: 2 (primer 2) were synthesized. The synthesis was made by Proligo Japan. By the PCR using this primer set, a 75 bp sequence in exon 7 of human G3PDH gene is amplified.
2. Preparation of PCR Reagents
   PCR reaction solutions were prepared from Takara Z-Taq (high-speed Taq DNA polymerase, Takara Bio Inc.,), and KOD Plus (KOD DNA polymerase, Toyo Boseki Kabushiki Kaisha, code KOD-201). As the reagents, everything except for bovine serum albumin (BSA, fraction V grade, SIGMA), primers, and a sample (human placenta DNA, SIGMA) came from the reagents attached with the polymerases, and the reagents were used in amounts as suggested by the protocol of the manufacturers. In (B), Z-Taq had been supplemented with 1 µg of anti-Taq DNA polymerase antibody (Toyo Boseki Kabushiki Kaisha, code TCP-101) for each 5U of Z-Taq. KOD Plus has been supplemented with anti-Taq DNA polymerase antibody to suppress non-specific reactions at low temperatures (hot start PCR). BSA was used to prevent DNA or enzymes from adhering to the glass capillaries.
   (A) Z-Taq (final amount 10 µL)
      - 2.5 mg/mL BSA:: 1 µL
      - Primer 1:: 2 pmol
      - Primer 2:: 2 pmol
      - x 10 Z-Taq buffer:: 1 µL
      - 2.5 mM dNTPs :: 0.8 µL
      - Z-Taq:: 0.25 U
      - Human DNA (2 ng/µL):: 1 µL
   (B) Z-Taq + anti-Taq antibody (final amount 10 µL)
      - 2.5 mg/mL:: BSA 1 µL
      - Primer 1:: 2 pmol
      - Primer 2:: 2 pmol
      - x 10 Z-Taq buffer:: 1 µL
      - 2.5 mM dNTPs:: 0.8 µL
      - Z-Taq (+antibody):: 0.25 U
      - Human DNA (2 ng/µL):: 1 µL
   (C) KOD-Plus (final amount 10 µL)
      - 2.5 mg/mL BSA:: 1 µL
      - Primer 1:: 2 pmol
      - Primer 2:: 2 pmol
      - x 10 KOD Plus buffer:: 1 µL
      - 2 mM dNTPs:: 1 µL
      - 25 mM MgSO₄:: 1.2 µL
      - KOD Plus:: 0.2 U
      - Human DNA (2 ng/µL):: 1 µL
3. PCR
   PCR was performed with a prototype high-speed PCR device (PCRJet) purchased from Megabase of the United States. The reaction was started from the denature step at 95°C for 15 seconds (in (B) and (C), the antibody comes off to activate DNA polymerase), and this was followed by 40 cycles of PCR under the following conditions.
   (1) 40 cycles: a total of about 10 minutes and 20 seconds 95°C for 0.2 seconds, 60°C for 5 seconds, 72°C for 5 seconds
   (2) 40 cycles: a total of about 8 minutes and 10 seconds 95°C for 0.2 seconds, 60°C for 5 seconds, 72°C for 2 seconds
   (3) 40 cycles: a total of about 6 minutes and 50 seconds 95°C for 0.2 seconds, 60°C for 5 seconds
   (4) 40 cycles: a total of about 6 minutes and 10 seconds 95°C for 0.2 seconds, 60°C for 4 seconds
   (5) 40 cycles: a total of about 5 minutes and 30 seconds 95°C for 0.2 seconds, 60°C for 3 seconds
   (6) 40 cycles: a total of about 4 minutes and 50 seconds 95°C for 0.2 seconds, 60°C for 2 seconds
4. Polyacrylamide Gel Electrophoresis
   Using 5% polyacrylamide gel, electrophoresis was performed at 100 V for 30 minutes in TBE buffer. The samples were stained with ethidium bromide and photographed under ultra violet light.
5. Results
   The results are shown in Figure 1. The two lanes on the both ends are size markers (øX174, degraded by HaeIII). PCR cycle conditions (1-6) and PCR reaction solutions (A-C) are as follows.
(Lanes from the left)
Marker
1-A
1-B
1-C
2-A
2-B
2-C
3-A
3-B
3-C
4-A
4-B
4-C
5-A
5-B
5-C
6-A
6-B
6-C
Marker

Table 1 below summarizes the results shown in Figure 1.

**[Table 1]**

| Cycle Conditions (40 cycles) | (1) | (2) | (3) | (4) | (5) | (6) |
|---|---|---|---|---|---|---|
| | 95°C 0.2 sec | 95°C 0.2 sec | 95°C 0.2 sec | 95°C 0.2 sec | 95°C 0.2 sec | 95°C 0.2 sec |
| | 60°C 5 sec | 60°C 5 sec | 60°C 5 sec | CO°C 4 sec | 60°C 3 sec | 60°C 2 sec |
| | 72°C 5 sec | 72°C 2 sec | | | | |
| Total Time | 10' 19 | 8' 11 | 6'50 | 6' 10 | 5' 31 | 4'54 |
| (A) Z-taq | ? (Ex) | ? (Ex) | ? (Ex) | ? (Ex) | - | - |
| (B) Z-taq + Antibody | ++ | + | + | - | - | - |
| (C) KOD Plus | ++ | ++ | ++ | + | + | - |

| | | | | | | |
|---|---|---|---|---|---|---|
| Ex: extra band | | | | | | |

In the sole Z-Taq sample (A), an extra band presumably due to a primer dimer was generated, which made it difficult to distinguish the target band. In sample (B) supplemented with anti-Taq antibody, no extra band was observed. The target band was clearly observed in cycle (1), but it was hardly recognizable in cycle (3) and unrecognizable in shorter cycles. In KOD Plus sample (C), the target band was clearly recognizable in cycle (3) and weakly recognizable even in cycle (5), confirming that amplification was possible in shorter cycles as compared with Z-Taq. Compared with the anneal/extension time of at least 7 seconds (10 seconds for sufficient amplification) required for Z-Taq, KOD Plus required only at least 3 seconds (5 seconds for sufficient amplification) for amplification. As a result, PCR was realized that required only a little more than 5 minutes to complete.

### INDUSTRIAL APPLICABILITY

The present invention enables detection and quantification of bacteria and viruses, quantification of RNA, and typing of single nucleotide polymorphism, both quickly and accurately. The present invention is therefore industrially highly useful in a wide variety of applications, including field environmental testing, clinical diagnosis at bedside, and urgent food inspection, for example.

## Claims

1. A method for performing a high-speed PCR under a temperature change of 10°C/sec or greater, the method comprising using a heat-resistant DNA polymerase having a deoxyribonucleic acid synthesis rate of 100 bases/sec or greater.

2. The method as set forth in claim 1, wherein the heat-resistant DNA polymerase originates in *Thermococcus.*

3. The method as set forth in claim 1, wherein the heat-resistant DNA polymerase originates in *Thermococcus kodakaraensis.*

4. A composition for high-speed PCR that is performed under a temperature change of 10°C/sec or greater, the composition comprising a heat-resistant DNA polymerase having a deoxyribonucleic acid synthesis rate of 100 bases/sec or greater.

5. A reagent kit for high-speed PCR that is performed under a temperature change of 10°C/sec or greater, the reagent kit comprising a heat-resistant DNA polymerase having a deoxyribonucleic acid synthesis rate of 100 bases/sec or greater.
